(19) Europäisches Patentamt / European Patent Office / Office européen des brevets

(11) **EP 3 858 940 A1**

(12) **EUROPEAN PATENT APPLICATION**
published in accordance with Art. 153(4) EPC

(43) Date of publication:
**04.08.2021 Bulletin 2021/31**

(51) Int Cl.:
*C09K 3/00* (2006.01)    *A61K 8/34* (2006.01)
*A61K 8/9789* (2017.01)    *A61K 8/99* (2017.01)
*A61Q 17/04* (2006.01)    *G02B 5/22* (2006.01)
*G02C 7/10* (2006.01)

(21) Application number: **19867378.2**

(22) Date of filing: **24.09.2019**

(86) International application number:
**PCT/JP2019/037374**

(87) International publication number:
**WO 2020/067061 (02.04.2020 Gazette 2020/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.09.2018 JP 2018185299**

(71) Applicant: **Murata Manufacturing Co., Ltd.**
**Nagaokakyo-shi, Kyoto 617-8555 (JP)**

(72) Inventors:
• **TAKATA, Masachika**
 **Nagaokakyo-shi, Kyoto 617-8555 (JP)**
• **SUNAHARA, Hirofumi**
 **Nagaokakyo-shi, Kyoto 617-8555 (JP)**

(74) Representative: **Reeve, Nicholas Edward**
 **Reddie & Grose LLP**
 **The White Chapel Building**
 **10 Whitechapel High Street**
 **London E1 8QS (GB)**

(54) **ELECTROMAGNETIC WAVE ABSORBER, SUNSCREEN, OPTICAL COMPONENT, EYEGLASSES, AND METHOD FOR PRODUCING ELECTROMAGNETIC WAVE ABSORBER**

(57) An electromagnetic wave absorber, comprising a polyphenol and lactic acid bacteria, wherein the absorber absorbs at least ultraviolet rays.

Fig. 1

Figure 1

EP 3 858 940 A1

## Description

Technical Field

The present invention relates to an electromagnetic wave absorber, a sunscreen agent, an optical component, a pair of eyeglasses, and a method for manufacturing the electromagnetic wave absorber.

Background Art

The sun light has ultraviolet rays (UV). The wavelength range of the ultraviolet rays is considered to be from approximately 100 nm or more and 400 nm or less, although this definition has minor differences depending on organizations and the like. The ultraviolet rays are classified, based on differences in biological effects due to wavelength difference, into ultraviolet A rays (UV-A), ultraviolet B rays (UV-B), and ultraviolet C rays (UV-C). The ultraviolet A rays are also called ultraviolet A region or long wavelength ultraviolet rays and the wavelength range is from 315 nm or more and 400 nm or less. The ultraviolet B rays are also called ultraviolet B region or medium wavelength ultraviolet rays and the wavelength range is from 280 nm or more and less than 315 nm. The ultraviolet C rays are also called ultraviolet C region or short wavelength ultraviolet rays and the wavelength range is from 100 nm or more and less than 280 nm.

The ultraviolet A rays penetrate deep into the skin, the dermis, and cause wrinkles, sagging as well as both reddish suntan and dark suntan called sunburns. Additionally, the ultraviolet A rays are absorbed by various biomolecules, give oxidative damages to membrane lipids, proteins, and DNA through active oxygen produced as a result of the absorption, and are involved with skin aging. The ultraviolet B rays have shallow penetration into the skin but have significant effects on the skin surface causing spots, wrinkles, as well as both sunburns and the subsequent dark suntan. Further, the ultraviolet B rays are absorbed in DNA present in the cell nucleus, give damages to DNA and cause skin cancers.

The shorter a wavelength is, the more significant damages ultraviolet rays cause to the biological materials, while the shorter a wavelength is, the more such rays tend to be absorbed in oxygen and the ozone layer. The ultraviolet C rays are absorbed in oxygen and the ozone layer and hardly observed on the ground. The ultraviolet B rays are also absorbed in oxygen and the ozone layer but partially reach the ground. The ultraviolet A rays mostly reach the ground. The ultraviolet A rays make up the majority of the ultraviolet rays which reach the ground. For this reason, the damages to the biological materials caused by the ultraviolet A rays are not negligible. For example, the contribution ratio to sunburn is 70% to 80% for the ultraviolet rays B and 20% to 30% for the ultraviolet A rays.

Conventional sunscreen agents contain organic compounds such as oxybenzone and avobenzone which absorb the ultraviolet A rays. However, these organic compounds are considered to have risks of causing skin inflammation and rough skin. To the contrary, Patent Literature 1 describes an ultraviolet ray absorber comprising the genus *Methylobacterium* and having an absorption peak of the ultraviolet ray absorption spectrum in a wavelength range from 320 nm to 400 nm. Patent Literature 2 discloses an electromagnetic wave absorber which absorbs at least ultraviolet rays and comprises the genus *Lactobacillus.* Patent Literature 3 mentions that a sprouted adlay fermentation product absorbs ultraviolet rays but the actual absorbance is unclear.

Citation List

Patent Literature

   Patent Literature 1: Japanese Patent No. 5751517
   Patent Literature 2: Japanese Patent Laid-Open No. 2017-222743
   Patent Literature 3: Japanese Patent Laid-Open No. 2007-290998

Summary of Invention

Technical Problem

A demand is made on an ultraviolet ray absorber which has low risks of causing skin inflammation and rough skin and further has a high ultraviolet ray absorption ability. The present invention was made under such circumstances and has an object to provide an electromagnetic wave absorber, a sunscreen agent, an optical component, and a pair of eyeglasses which have low risks of causing skin inflammation and rough skin and are capable of efficiently absorbing ultraviolet rays, and a method for manufacturing the electromagnetic wave absorber.

Solution to Problem

The electromagnetic wave absorber according to an aspect of the present invention absorbs at least ultraviolet rays and contains a polyphenol and lactic acid bacteria. The sunscreen agent according to an aspect of the present invention contains the above electromagnetic wave absorber. The optical component according to an aspect of the present invention includes a transparent member and the above electromagnetic wave absorber arranged on the transparent member. Further, the pair of eyeglasses according to an aspect of the present invention includes the above optical component as lenses. Furthermore, a method for manufacturing an electromagnetic wave absorber which absorbs at least ultraviolet rays according to an aspect of the present invention includes mixing a polyphenol and lactic acid bacteria.

Advantageous Effects of Invention

According to the present invention, an electromagnetic wave absorber, a sunscreen agent, an optical component, and a pair of eyeglasses which have low risks of causing skin inflammation and rough skin and are capable of efficiently absorbing ultraviolet rays, and a method for manufacturing the electromagnetic wave absorber can be provided.

Brief Description of Drawings

[Figure 1] Figure 1 is an optical spectrum showing measurement results of absorbances according to Example 4.
[Figure 2] Figure 2 is a table showing measurement results of absorbances according to Example 5.
[Figure 3] Figure 3 is a table showing measurement results of absorbances according to Example 5.

Description of Embodiments

Hereinafter, embodiments of the present invention are described in detail. The following embodiments are to illustrate apparatuses and methods to embody the technical ideas of the present invention and the technical ideas of the present invention do not limit combinations of the component members and the like to the following descriptions. The technical ideas of the present invention can have various modifications within the scope of claims.

(First embodiment)

The electromagnetic wave absorber according to the first embodiment contains a polyphenol and lactic acid bacteria and absorbs at least ultraviolet rays. Examples of the polyphenol include chlorogenic acids, catechin, anthocyanin, tannin, rutin, isoflavone, ellagic acid, lignan, curcumin, and coumarin but are not limited thereto. Chlorogenic acids are ester compounds of cinnamic acid derivatives and quinic acid. The polyphenol may be naturally-derived or an artificially synthetic compound which has been confirmed to be safe.
The polyphenol is derived from, for example, the family *Apiaceae* or *Asteraceae.* Examples of the family *Apiaceae* include the genus *Angelica.* Examples of the genus *Angelica* include *Angelica keiskei, Angelica decursiva, Angelica pubescens, Angelica dahurica,* and Japanese angelica. Examples of the family *Asteraceae* include the genus *Artemisia.* Examples of the genus *Artemisia* include *Artemisia indica* var. *maximowiczii, Artemisia montana,* worm wood, and tarragon. The electromagnetic wave absorber according to the embodiment may contain one or more polyphenols. Examples of the chlorogenic acids contained in the families *Apiaceae* and *Asteraceae* include 3,5-dicaffeoylquinic acid, 3,4-dicaffeoylquinic acid, and 4,5-dicaffeoylquinic acid but are not limited thereto.
Examples of the lactic acid bacteria include the genera *Lactobacillus, Lactococcus, Enterococcus, Pediococcus, Leuconostoc, Streptococcus,* and *Bifidobacterium* but are not limited thereto. The electromagnetic wave absorber according to the embodiment may contain one or more species of lactic acid bacteria.
The electromagnetic wave absorber according to the embodiment may contain a mixture of the polyphenol and the lactic acid bacteria in a solution. Alternatively, the electromagnetic wave absorber according to the embodiment may contain a dried powder of the mixture of the polyphenol and the lactic acid bacteria. It is preferable that the polyphenol attach to the lactic acid bacteria. The lactic acid bacteria may be dead bacteria, and the mixture of the polyphenol and the lactic acid bacteria may be heat treated. Heat treatment of the mixture of the polyphenol and the lactic acid bacteria promotes the attachment of the polyphenol to the lactic acid bacteria. For example, the lactic acid bacteria to which the polyphenol does not attach are white but the lactic acid bacteria to which the polyphenol attaches are yellow or brown. Thus, microscopic observation of the lactic acid bacteria enables the confirmation on the attachment of the polyphenol to the lactic acid bacteria. Heat treatment of the mixture of the polyphenol and the lactic acid bacteria further increases electromagnetic wave absorption properties of the electromagnetic wave absorber according to the embodiment.
The lactic acid bacteria to which the polyphenol attaches may coagulate with each other. The lactic acid bacteria to which the polyphenol attaches coagulate with each other by, for example, a hydrophobic bond. Coagulation of the lactic acid bacteria to which the polyphenol attaches with each other further increases electromagnetic wave absorption properties of the electromagnetic wave absorber according to the embodiment.
The electromagnetic wave absorber according to the embodiment absorbs at least a part of the wavelength ranges of ultraviolet rays and visible light. For example, the wavelength range of ultraviolet rays the electromagnetic wave absorber according to the embodiment absorbs is from 200 nm or more and 400 nm or less. The wavelength range of the ultraviolet A rays the electromagnetic wave absorber according to the embodiment absorbs is from 315 nm or more and 400 nm or less. The wavelength range of the ultraviolet B rays the electromagnetic wave absorber according to the embodiment absorbs is from 280 nm or more and 315 nm or less. The wavelength range of the ultraviolet C rays the electromagnetic wave absorber according to the embodiment absorbs is from 200 nm or more and 280 nm or less.
The wavelength range of visible light the electromagnetic wave absorber according to the embodiment absorbs is from 400 nm or more, 800 nm or less, 700 nm or less, 650 nm or less, or 600 nm or less. The wavelength range of visible light the electromagnetic wave absorber according to the embodiment absorbs includes blue light including high energy visible light of from 400 nm or more and 500 nm or less.

The electromagnetic wave absorber according to the embodiment tends to absorb light in a shorter wavelength visible range nearer to ultraviolet rays than in a longer wavelength visible range and thus tends to absorb purple and blue lights and allow red and green lights to transmit. For this reason, the transmitted light of the electromagnetic wave absorber according to the embodiment tends to look yellow to brown.

The electromagnetic wave absorber according to the first embodiment contains the lactic acid bacteria in a concentration of, for example, 0.0001 wt% or more, 0.0005 wt% or more, 0.001 wt% or more, 0.005 wt% or more, or 0.01 wt% or more. The electromagnetic wave absorber according to the first embodiment may contain the lactic acid bacteria in an even higher concentration. However, the electromagnetic wave absorber according to the first embodiment can absorb ultraviolet rays even when the concentration of the lactic acid bacteria is as low as less than 0.001 wt%. The content of the lactic acid bacteria in the electromagnetic wave absorber according to the first embodiment is suitably determined in accordance with an intended ultraviolet ray absorption amount. It should be noted that Bacterial cells are removed from conventional *Artemisia indica* var. *maximowiczii* creams, *Angelica keiskei* creams and the like or they do not substantially contain bacterial cells.

As for the lactic acid bacteria, for example, heat-treated dead bacteria, tend to absorb more ultraviolet rays than live bacteria. Thus, the electromagnetic wave absorber according to the first embodiment may contain dead lactic acid bacteria. The lactic acid bacteria may be dried bacterium. Drying the lactic acid bacteria enables stable storage for an extended period of time.

The ultraviolet rays having longer wavelengths tend to more easily penetrate the skin. Similarly, in the range of ultraviolet A rays, the ultraviolet rays having longer wavelengths tend to more easily penetrate the skin. To the contrary, the electromagnetic wave absorber according to the first embodiment provides an effect to prevent the ultraviolet rays from penetrating the skin due to the ultraviolet ray absorption effect over wide wavelength ranges. Thus, the electromagnetic wave absorber according to the first embodiment can be applied externally on the skin or hair and utilized as a sunscreen agent to prevent inflammation caused by sunburn. Additionally, the electromagnetic wave absorber according to the first embodiment containing a polyphenol and lactic acid bacteria as active ingredients has little burden on the skin.

The polyphenol and the lactic acid bacteria are safe in the body and thus even when the electromagnetic wave absorber according to the first embodiment is accidentally ingested by a toddler or an elderly person, it is safe.

The electromagnetic wave absorber according to the first embodiment may be added to skin care cosmetic products such as a toner, an emulsion, a cream, a spray, and a lotion. Further, the electromagnetic wave absorber according to the first embodiment may be added to make-up cosmetics such as a primer, a foundation, a rouge, a face color and an eyeliner, and the like.

The electromagnetic wave absorber according to the embodiment may be contained in, for example, skin conditioning cosmetics. Examples of the skin conditioning cosmetic include toners, lotions, and face packs. The electromagnetic wave absorber according to the embodiment may be contained in, for example, skin protecting cosmetics. Examples of the skin protecting cosmetic include skin protecting emulsions and skin protecting creams. The electromagnetic wave absorber according to the embodiment may be contained in, for example, make-up base cosmetics. Examples of the make-up base cosmetic include foundations, facial powders, and make-up bases. The electromagnetic wave absorber according to the embodiment may be contained in, for example, point make-up cosmetics. Examples of the point make-up cosmetic include rouges, eye make-ups, blushers, and nail enamels.

The electromagnetic wave absorber according to the first embodiment may suitably contain, in addition to the polyphenol and the lactic acid bacteria, additional ingredients of cosmetics and medicaments in accordance with the purpose such as a color, a liquid fat and oil, a solid fat and oil, a wax, a hydrocarbon, a higher fatty acid, a higher alcohol, an ester, a silicone, an anionic surfactant, a cationic surfactant, an amphoteric surfactant, a nonionic surfactant, a moisturizer, a water-soluble polymer, a thickener, a coating agent, a sequestering agent, a lower alcohol, a polyhydric alcohol, a saccharide, an amino acid, an organic amine, a pH adjusting agent, a skin nutrient, a vitamin, an antioxidant, a perfume, a powder, a coloring material, and water.

When the electromagnetic wave absorber according to the embodiment is contained in a cosmetic containing an oily component, a concentration of the oily component in the cosmetic is not particularly limited but, for example, 0.1 mass% or more and 90 mass% or less, or 0.5 mass% or more and 90 mass% or less. When the electromagnetic wave absorber according to the embodiment is contained in a cosmetic containing an aqueous component, a concentration of the aqueous component in the cosmetic is not particularly limited but, for example, 0.1 mass% or more and 90 mass% or less, or 0.5 mass% or more and 90 mass% or less. The ratio of an oily component and an aqueous component in a cosmetic is suitably determined whether the cosmetic is an oil-in-water (O/W) type or a water-in-oil (W/O) type. When the electromagnetic wave absorber according to the embodiment is contained in a cosmetic containing a surfactant, a concentration of the surfactant in the cosmetic is not particularly limited but, for example, 2 mass% or more and 10 mass% or less.

Further, the electromagnetic wave absorber according to the first embodiment may suitably contain, in addition to the polyphenol and the lactic acid bacteria, an organic compound ultraviolet ray absorber, an inorganic compound ultraviolet ray scattering agent or the like in accordance with purposes. For example, an ultraviolet ray absorber which does not absorb the ultraviolet A rays and the electromagnetic wave absorber according to the first embodiment which absorbs the ultraviolet A rays may be used in combination.

The electromagnetic wave absorber according to the first embodiment is manufactured by mixing the polyphenol and the lactic acid bacteria. The polyphenol and the lactic acid bacteria may be mixed in a solvent to form a mixture solution. By mixing the polyphenol and the lactic acid bacteria, the polyphenol attaches to the lactic acid bacteria. The mixture of the polyphenol and the lactic acid bacteria may be heated. A heating temperature is, for example, 100°C or more, 110°C or more, or 120°C or more. The heating promotes the attachment of the polyphenol to the lactic acid bacteria.

The mixture of the polyphenol and the lactic acid bacteria may be collected from the mixture solution of the polyphenol and the lactic acid bacteria by centrifugation or the like. The collected mixture of the polyphenol and the lactic acid bacteria may be dried to obtain a dried powder of the mixture of the polyphenol and the lactic acid bacteria. Further, the dried powder of the mixture of the polyphenol and the lactic acid bacteria may be added to a solvent to form a solution containing the mixture of the polyphenol and the lactic acid bacteria. In the manufactured electromagnetic wave absorber, the final concentration of the lactic acid bacteria may be less than 0.001 wt%.

(Second embodiment)

In the second embodiment and thereafter, descriptions on the common matters with the first embodiment are omitted and only different aspects will be described. In particular, no reference will be made in each embodiment on the same functional effects provided by the same structure.

The electromagnetic wave absorber containing a polyphenol and lactic acid bacteria may be added as an anti-ultraviolet ray material to products other than skin external agents, such as a paint, a dye, a pigment, a resin, a synthetic rubber, a latex, a film, and a fiber.

For example, the optical component according to the second embodiment includes a transparent member and a coating layer, which is coated on the transparent member and to which the electromagnetic wave absorber described in the first embodiment is added. The transparent member is made of a transparent material such as a glass or a resin. The optical component according to the second embodiment can be utilized as, for example, lenses for a pair of eyeglasses and a transparent panel for televisions and computers.

The electromagnetic wave absorber containing the polyphenol and the lactic acid bacteria absorbs blue light including high energy visible light as described in the first embodiment and the optical component according to the second embodiment hence prevents not only the ultraviolet rays but also blue light from entering the eyes. For this reason, such an optical component has an effect to suppress eyestrain. Additionally, the electromagnetic wave absorber according to the second embodiment absorbs, but does not reflect, and the optical component thereby causes no bluishness.

As described above, the electromagnetic wave absorber or the like according to each embodiment of the present invention and the like has a composition and an operational advantage as illustrated below by any one of or a combination of the described above.

The electromagnetic wave absorber according to the present embodiments contains a polyphenol and lactic acid bacteria. The electromagnetic wave absorber according to the present embodiments can absorb at least ultraviolet rays. Further, the electromagnetic wave absorber according to the present embodiments can also absorb blue light.

The electromagnetic wave absorber according to the present embodiments may contain a heated mixture of the polyphenol and the lactic acid bacteria. The polyphenol may attach to the lactic acid bacteria. The lactic acid bacteria to which the polyphenol attaches may coagulate with each other. Such a coagulation improves the ultraviolet ray absorption ability.

The electromagnetic wave absorber according to the present embodiments may contain the lactic acid bacteria in a concentration of less than 0.001 wt%. The electromagnetic wave absorber according to the present embodiments can absorb ultraviolet rays even when the concentration of the lactic acid bacteria is low.

The electromagnetic wave absorber according to the present embodiments can contain a mixture solution of the polyphenol and the lactic acid bacteria or may contain a powder of the polyphenol and the lactic acid bacteria. The electromagnetic wave absorber according to the present embodiments, in either form, can absorb ultraviolet rays.

In the electromagnetic wave absorber according to the present embodiments, the polyphenol may include chlorogenic acids. The polyphenol may be derived from the family *Apiaceae* or the family *Asteraceae*. The family *Apiaceae* may include *Angelica keiskei*. The family *Asteraceae* may include *Artemisia indica* var. *maximowiczii*. The lactic acid bacteria may be from at least any one selected from the group consisting of the genera *Lactobacillus, Lactococcus, Enterococcus, Pediococcus, Leuconostoc, Streptococcus,* and *Bifidobacterium.* Regardless the types of the polyphenol and the lactic acid bacteria, the electromagnetic wave absorber according to the present embodiments can absorb ultraviolet rays.

The sunscreen agent according to the present embodiments contains the above electromagnetic wave absorber. The sunscreen agent according to the present embodiments is safe to the human body and can absorb ultraviolet rays.

The optical component according to the present embodiments includes a transparent member and the above electromagnetic wave absorber arranged on the transparent member. The pair of eyeglasses according to the present embodiments includes the above optical component as lenses. The optical component and the pair of eyeglasses according to the present embodiments can absorb ultraviolet rays.

A method for manufacturing the electromagnetic wave absorber according to the present embodiments comprises mixing the polyphenol and the lactic acid bacteria. The electromagnetic wave absorber manufactured by the manufacturing method according to the present embodiments can absorb at least ultraviolet rays.

The method for manufacturing the electromagnetic wave absorber according to the present embodiments may further comprise heating the polyphenol and the lactic acid bacteria. Heating may be carried out at 100°C or more. The polyphenol may attach to the lactic acid bacteria. The lactic acid bacteria to which the polyphenol attach may coagulate with each other. Such a coagulation improves the ultraviolet ray absorption ability of the electromagnetic wave absorber to be manufactured.

In the method for manufacturing the electromagnetic wave absorber according to the present embodiments, the final concentration of the lactic acid bacteria may be less than 0.001 wt%. The electromagnetic wave absorber to be manufactured can absorb ultraviolet rays even when the concentration of the lactic acid bacteria is low.

In the method for manufacturing the electromagnetic wave absorber according to the present embodiments, a mixture solution of the polyphenol and the lactic acid bacteria may be manufactured in mixing. The method may further comprise manufacturing a powder of the mixture of the polyphenol and the lactic acid bacteria. The electromagnetic wave absorber to be manufactued, in either form, can absorb ultraviolet rays.

In the method for manufacturing the electromagnetic wave absorber according to the present embodiments, the polyphenol may include chlorogenic acids. The polyphenol may be derived from the family *Apiaceae* or *Asteraceae.* The family *Apiaceae* may include *Angelica keiskei.* The family *Asteraceae* may include *Artemisia indica* var. *maximowiczii.* The lactic acid bacteria may be from at least any one selected from the group consisting of the genera *Lactobacillus, Lactococcus, Enterococcus, Pediococcus, Leuconostoc, Streptococcus,* and *Bifidobacterium.* Regardless the types of the polyphenol and the lactic acid bacteria, the electromagnetic wave absorber to be manufactured can absorb ultraviolet rays.

Examples

Examples of the present invention are described below. However, the present invention is, needless to say, not limited to the following examples.

(Example 1: Extraction of chlorogenic acids from *Artemisia indica* var. *maximowiczii*)

*Artemisia indica* var. *maximowiczii* was dried at 50°C for 3 days. The dried *Artemisia indica* var. *maximowiczii* was powdered using a grinder. Then, solvent-soluble components of the powdered *Artemisia indica* var. *maximowiczii* were eluted in ethyl acetate using a Soxhlet extractor to obtain an *Artemisia indica* var. *maximowiczii* component solution. Subsequently, ethyl acetate was removed from the *Artemisia indica* var. *maximowiczii* component solution using an evaporator thereby to extract *Artemisia indica* var. *maximowiczii* components containing mainly polyphenols such as chlorogenic acids. Note that this method did not comprise a fermentation step.

(Example 2: Extraction of chlorogenic acids from *Angelica keiskei)*

*Angelica keiskei* components containing mainly polyphenols such as chlorogenic acids were extracted from *Angelica keiskei* by the same method as in Example 1.

(Example 3: Obtention of lactic acid bacteria)

Strains of lactic acid bacteria, *Streptococcus thermophilus* (NBRC 13957), *Leuconostoc mesenteroides* (NBRC 110676), *Pediococcus damnosus* (NBRC 3889), *Lactococcus lactis subsp. lactis* (NBRC 12007), *Lactobacillus plantarum* (NBRC 3070), *Enterococcus faecalis* (NBRC 3971), and *Bifidobacterium bifidum* (NBRC 100015) were obtained from Biological Resource Center, NITE (NBRC).

(Example 4: Comparison of absorbances depending on sample preparation conditions)

A suspension containing *Lactobacillus plantarum* in water as solvent in a concentration of 0.0005 wt% was prepared as Sample 1. A suspension containing the *Artemisia indica* var. *maximowiczii* extraction components in water as solvent in a concentration of 0.0005 wt% was prepared as Sample 2. A suspension containing the *Artemisia indica* var. *maximowiczii* extraction components in a concentration of 0.0005 wt% and *Lactobacillus plantarum* in a concentration of 0.0005 wt% in water as solvent was prepared as Sample 3. Sample 3 was heat treated at 121°C for 20 minutes to prepare Sample 4. Sample 4 was centrifuged at 10000 rpm for 20 minutes to collect the bacteria to which polyphenols attached, and the collected bacteria were air dried to obtain the dried bacteria. A suspension in which the dried bacteria were suspended in a concentration of 0.0005 wt% in water as solvent was prepared as Sample 5.

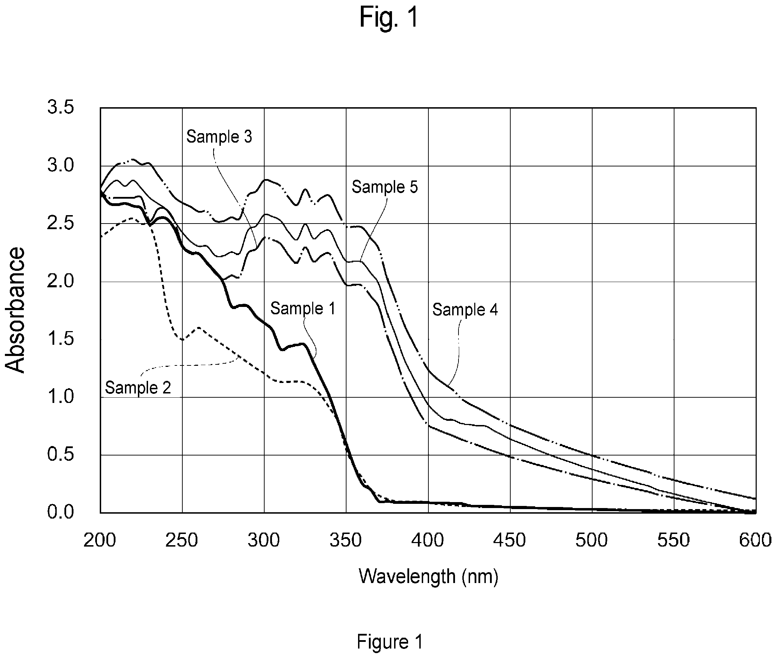

Absorbances of Samples 1 to 5 in wavelength ranges of 200 nm or more and 600 nm or less were measured using an ultraviolet and visible spectrophotometer (UV-1280, Shimadzu Corporation). As shown in Figure 1, Sample 3 containing the bacteria and the *Artemisia indica* var. *maximowiczii* extraction components, when compared with Sample 1 containing only the bacteria and Sample 2 containing only the *Artemisia indica* var. *maximowiczii* extraction components, had increased absorbance in the ranges of UV-A and UV-B and also had increased absorbance in the blue light range of visible light range. Further, the heat-treated Sample 4 and Sample 5, when compared with Sample 3, had further increased absorbances in the ranges of UV-A and UV-B and also had increased absorbances in the blue light range of visible light range. Note that the absorbance Abs is obtained by the following formula when a transmitted light intensity of pure water placed in a cell is $I_0$ and a transmitted light intensity of a sample solution place in a cell is $I_t$.

$$\text{Abs} = \text{Log}_{10} \ (I_0/I_t)$$

It is presumed, but not to be bound by any theory, that a phosphate and a carboxyl group on the surface of the lactic acid bacteria and the polyphenol such as chlorogenic acids interact by weak bonds caused by intermolecular forces such as hydrogen bonds in the solution whereby a complex of the lactic acid bacteria and the polyphenol is formed. The formation of the complex by the lactic acid bacteria and the polyphenol neutralizes respective electrical polarities of the lactic acid bacteria and the polyphenol. It is presumed that this decreases the numbers of water molecules capable of bonding to the lactic acid bacteria and the polyphenol, respectively and the complex of the lactic acid bacteria and the polyphenol has an increased hydrophobicity when compared with the lactic acid bacteria alone and the polyphenol alone.

The complexes of the lactic acid bacteria and the polyphenol bond together by hydrophobic interaction to form agglomerates. For this reason, it is presumed that Sample 3, when compared with Samples 1 and 2, had improved the UV absorption properties on the longer wavelength side.

Further, it is presumed that heat treatment of the complex of the lactic acid bacteria and the polyphenol causes the condensation reaction of the carboxyl group and the hydroxyl group of chlorogenic acids on the surface of the lactic acid bacteria thereby to produce a carbonyl group. This affects the conjugated double bond of chlorogenic acids whereby the UV absorption properties on the longer wavelength side is improved in the heat-treated complex of the lactic acid bacteria and the polyphenol. For this reason, it is presumed that Samples 4 and 5, when compared with Sample 3, had improved UV absorption properties on the longer wavelength side.

(Example 5: Comparison of absorbances by derivation of polyphenol and kind of lactic acid bacteria)

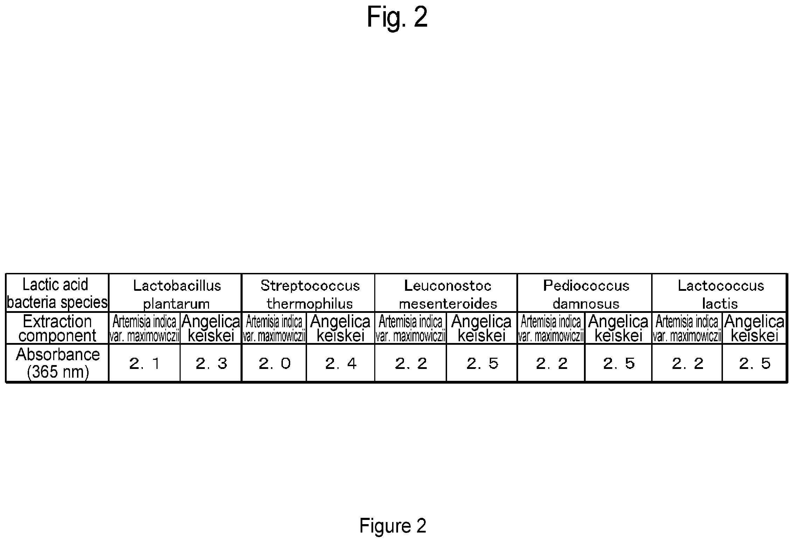

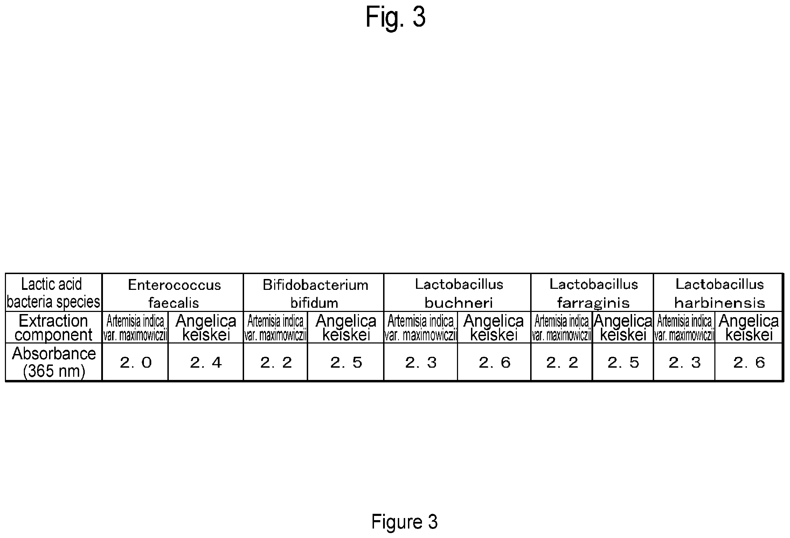

A suspension containing the *Artemisia indica* var. *maximowiczii* extraction components or the *Angelica keiskei* extraction components in a concentration of 0.0005 wt% and each of the lactic acid bacteria in a concentration of 0.0005 wt% in water as solvent was prepared. Further, the suspension was heat treated at 121°C for 20 minutes. Each of the heat-treated suspensions was measured for the absorbance at a wavelength of 365 nm. As a result, as shown in Figure 2 and Figure 3, good absorbances were obtained in any of the combinations.

Each of the embodiments and examples described above is to facilitate the understanding of the present invention and is not intended to interpret the present invention with limitations. The present invention can be altered/modified without departing from the spirit of the present invention and encompasses the equivalents thereof. That is, each of the embodiments and examples suitably altered in design by those skilled in the art is also encompassed in the scope of the present invention as long as it includes features of the present invention. For example, each element in each of the embodiments and examples is not limited to those illustrated and can be suitably altered. Further, each of the embodiments and examples is only an illustration and it is obvious that the structures described in different embodiments can be partially substituted or combined, and those are also encompassed in the scope of the present invention as long as they include features of the present invention.

**Claims**

1. An electromagnetic wave absorber, comprising a polyphenol and lactic acid bacteria, wherein the absorber absorbs at least ultraviolet rays.

2. The electromagnetic wave absorber according to claim 1, comprising a heated mixture of the polyphenol and the lactic acid bacteria.

3. The electromagnetic wave absorber according to claim 1 or 2, wherein the polyphenol attaches to the lactic acid bacteria.

4. The electromagnetic wave absorber according to claim 3, wherein the lactic acid bacteria to which the polyphenol attaches coagulate with each other.

5. The electromagnetic wave absorber according to any one of claims 1 to 4, comprising the lactic acid bacteria in a concentration of less than 0.001 wt%.

6. The electromagnetic wave absorber according to any one of claims 1 to 5, comprising a mixture solution of the polyphenol and the lactic acid bacteria.

7. The electromagnetic wave absorber according to any one of claims 1 to 5, comprising a powder of the polyphenol and the lactic acid bacteria.

8. The electromagnetic wave absorber according to any one of claims 1 to 7, wherein the polyphenol comprises chlorogenic acids.

9. The electromagnetic wave absorber according to any one of claims 1 to 8, wherein the polyphenol is derived from the family *Apiaceae* or *Asteraceae.*

10. The electromagnetic wave absorber according to claim 9, wherein the family *Apiaceae* includes *Angelica keiskei.*

11. The electromagnetic wave absorber according to claim 9, wherein the family *Asteraceae* includes *Artemisia indica* var. *maximowiczii.*

12. The electromagnetic wave absorber according to any one of claims 1 to 11, wherein the lactic acid bacteria are from at least any one selected from the group consisting of the genera *Lactobacillus, Lactococcus, Enterococcus, Pediococcus, Leuconostoc, Streptococcus,* and *Bifidobacterium.*

13. The electromagnetic wave absorber according to any one of claims 1 to 12, wherein the absorber absorbs a blue light.

14. A sunscreen agent comprising the electromagnetic wave absorber according to any one of claims 1 to 13.

15. An optical component comprising:

   a transparent member; and
   the electromagnetic wave absorber according to any one of claims 1 to 13 arranged on the transparent member.

16. A pair of eyeglasses, comprising the optical component according to claim 15 as lenses.

17. A method for manufacturing an electromagnetic wave absorber which absorbs at least ultraviolet rays, comprising mixing a polyphenol and lactic acid bacteria.

18. The method for manufacturing an electromagnetic wave absorber according to claim 17, further comprising heating the polyphenol and the lactic acid bacteria.

19. The method for manufacturing an electromagnetic wave absorber according to claim 18, wherein the heating is carried out at 100°C or more.

20. The method for manufacturing an electromagnetic wave absorber according to any one of claims 17 to 19, wherein the polyphenol attaches to the lactic acid bacteria.

21. The method for manufacturing an electromagnetic wave absorber according to claim 20, wherein the lactic acid bacteria to which the polyphenol attaches coagulate with each other.

22. The method for manufacturing an electromagnetic wave absorber according to any one of claims 17 to 21, wherein a final concentration of the lactic acid bacteria is less than 0.001 wt%.

23. The method for manufacturing an electromagnetic wave absorber according to any one of claims 17 to 22, wherein a mixture solution of the polyphenol and the lactic acid bacteria is manufactured in the mixing.

24. The method for manufacturing an electromagnetic wave absorber according to any one of claims 17 to 23, further comprising manufacturing a powder of a mixture of the polyphenol and the lactic acid bacteria.

25. The method for manufacturing an electromagnetic wave absorber according to any one of claims 17 to 24, wherein the polyphenol comprises chlorogenic acids.

26. The method for manufacturing an electromagnetic wave absorber according to any one of claims 17 to 25, wherein the polyphenol is derived from the family *Apiaceae* or *Asteraceae.*

27. The method for manufacturing an electromagnetic wave absorber according to claim 26, wherein the family *Apiaceae* includes *Angelica keiskei.*

28. The method for manufacturing an electromagnetic wave absorber according to claim 26, wherein the family *Asteraceae* includes *Artemisia indica* var. *maximowiczii.*

29. The method for manufacturing an electromagnetic wave absorber according to any one of claims 17 to 28, wherein the lactic acid bacteria are from at least any one selected from the group consisting of the genera *Lactobacillus, Lactococcus, Enterococcus, Pediococcus, Leuconostoc, Streptococcus,* and *Bifidobacterium.*

Fig. 1

Figure 1

EP 3 858 940 A1

# Fig. 2

| Lactic acid bacteria species | Lactobacillus plantarum | | Streptococcus thermophilus | | Leuconostoc mesenteroides | | Pediococcus damnosus | | Lactococcus lactis | |
|---|---|---|---|---|---|---|---|---|---|---|
| Extraction component | Artemisia indica var. maximowiczii | Angelica keiskei | Artemisia indica var. maximowiczii | Angelica keiskei | Artemisia indica var. maximowiczii | Angelica keiskei | Artemisia indica var. maximowiczii | Angelica keiskei | Artemisia indica var. maximowiczii | Angelica keiskei |
| Absorbance (365 nm) | 2. 1 | 2. 3 | 2. 0 | 2. 4 | 2. 2 | 2. 5 | 2. 2 | 2. 5 | 2. 2 | 2. 5 |

Figure 2

EP 3 858 940 A1

# Fig. 3

EP 3 858 940 A1

| Lactic acid bacteria species | Enterococcus faecalis | | Bifidobacterium bifidum | | Lactobacillus buchneri | | Lactobacillus farraginis | | Lactobacillus harbinensis | |
|---|---|---|---|---|---|---|---|---|---|---|
| Extraction component | Artemisia indica var. maximowiczii | Angelica keiskei | Artemisia indica var. maximowiczii | Angelica keiskei | Artemisia indica var. maximowiczii | Angelica keiskei | Artemisia indica var. maximowiczii | Angelica keiskei | Artemisia indica var. maximowiczii | Angelica keiskei |
| Absorbance (365 nm) | 2. 0 | 2. 4 | 2. 2 | 2. 5 | 2. 3 | 2. 6 | 2. 2 | 2. 5 | 2. 3 | 2. 6 |

Figure 3

## INTERNATIONAL SEARCH REPORT

| International application No. |
| --- |
| PCT/JP2019/037374 |

| A. CLASSIFICATION OF SUBJECT MATTER |
| --- |
| Int.Cl. C09K3/00(2006.01)i, A61K8/34(2006.01)i, A61K8/9789(2017.01)i, A61K8/99(2017.01)i, A61Q17/04(2006.01)i, G02B5/22(2006.01)i, G02C7/10(2006.01)i |
| According to International Patent Classification (IPC) or to both national classification and IPC |

| B. FIELDS SEARCHED |
| --- |
| Minimum documentation searched (classification system followed by classification symbols) |
| Int.Cl. C09K3/00, A61K8/34, A61K8/9789, A61K8/99, A61Q17/04, G02B5/22, G02C7/10 |

| Documentation searched other than minimum documentation to the extent that such documents are included in the fields searched |
| --- |
| Published examined utility model applications of Japan 1922–1996 |
| Published unexamined utility model applications of Japan 1971–2019 |
| Registered utility model specifications of Japan 1996–2019 |
| Published registered utility model applications of Japan 1994–2019 |

| Electronic data base consulted during the international search (name of data base and, where practicable, search terms used) |
| --- |
| |

### C. DOCUMENTS CONSIDERED TO BE RELEVANT

| Category* | Citation of document, with indication, where appropriate, of the relevant passages | Relevant to claim No. |
| --- | --- | --- |
| X<br>Y<br>A | JP 2017-222743 A (MURATA MANUFACTURING CO., LTD.) 21 December 2017, claims 1, 10-11, 14-20, paragraphs [0005]-[0007], [0020], examples 1-4, fig. 7, 8<br>(Family: none) | 1-16<br>17-18, 20-29<br>19 |
| Y<br>A | JP 8-151319 A (T HASEGAWA CO., LTD.) 11 June 1996, claim 1, paragraphs [0004], [0005]<br>(Family: none) | 17-18, 20-29<br>1-16, 19 |

☒ Further documents are listed in the continuation of Box C.    ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 28.10.2019 | 05.11.2019 |

| Name and mailing address of the ISA/ | Authorized officer |
| --- | --- |
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.

PCT/JP2019/037374

C (Continuation). DOCUMENTS CONSIDERED TO BE RELEVANT

| Category* | Citation of document, with indication, where appropriate, of the relevant passages | Relevant to claim No. |
|---|---|---|
| A | WO 2009/041640 A1 (TAKASAGO INTERNATIONAL CORPORATION) 02 April 2009, paragraph [0017] & US 2010/0210866 A1, paragraph [0045] & EP 2196208 A1 & CN 101808653 A | 1-29 |
| A | JP 2010-235470 A (LION CORPORATION) 21 October 2010, paragraphs [0010], [0011] (Family: none) | 1-29 |
| A | JP 2015-525800 A (TOPGENIX, INC.) 07 September 2015, claims 7, 12, 22-23 & US 2014/0044653 A1, claims 2, 9 & WO 2014/025938 A1 & EP 2882494 A1 & AU 2013299605 A & KR 10-2015-0065170 A & IL 237149 A & MX 2015001751 A | 1-29 |
| A | JP 8-92057 A (ICHIMARU PHARCOS CO., LTD.) 09 April 1996, claims 1-3, paragraph [0013] (Family: none) | 1-29 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5751517 B
- JP 2017222743 A
- JP 2007290998 A